# EUROPEAN PATENT SPECIFICATION

(11) **EP 2 756 793 B1**
(45) Date of publication and mention of the grant of the patent: **28.11.2018**
(21) Application number: 14020002.3
(22) Date of filing: 09.01.2014
(51) Int. Cl.: A61B 1/24

(54) **Autocapture for intra-oral imaging using inertial sensing**
Automatische Erfassung für intraorale Bilderzeugung mittels Trägheitsmessung
Capture automatique d'imagerie intra-orale utilisant une détection inertielle

(30) Priority: 16.01.2013 US 201361753001 P; 14.03.2013 US 201313803489
(43) Date of publication of application: 23.07.2014
(73) Proprietor: Carestream Dental Technology Topco Limited, London SW1Y 6RJ (GB)
(72) Inventor: Apte, Pushkar, San Jose, CA 95136 (US); Milch, James R., Penfield, NY 14526 (US)
(74) Representative: Carstens, Dirk Wilhelm

(56) References cited:
- JP-A- 2012 075 690
- US-A1- 2005 236 488
- US-A1- 2010 268 069

## Description

### FIELD OF THE INVENTION

The invention relates generally to the field of intra-oral imaging and more particularly relates to methods and apparatus for initiation and control of the image capture sequence for three-dimensional (3D) intra-oral imaging using inertial sensing.

### BACKGROUND

Three-dimensional (3D) intra-oral imaging provides a tool for the dental practitioner, enabling volume imaging information to be displayed and stored. The information that is obtained from a 3D model can be used for a range of purposes related to diagnosis and treatment and can be useful when preparing a crown, implant, or other restorative structure.

3D measurement and imaging reconstructs surface information from a set of two-dimensional (2D) images obtained from the patient. The imaging sequence can also include acquiring images of a pattern of lines or other features projected onto the teeth or other surface and correlated with 2D image content. In order to form or reconstruct the 3D model from 2D images, the reconstruction algorithms must be able to correlate the individual 2D images to each other so that pixel positions and vertex coordinates can be accurately identified. This requires that the relative position and orientation of the camera when capturing each image be identified and requires that camera movement from one position to another be carefully tracked. This can be particularly difficult when imaging larger portions of the tooth arch, for example.

Among the problems with obtaining the set of images needed for 3D reconstruction is obtaining the images with appropriate timing. This requires, for example, that the camera be at rest before the image capture sequence begins and also that movement of the camera during the image capture interval should be minimal and that this movement should be tracked.

Some approaches to intra-oral 3D imaging require the operator or technician to manually initiate and control the image capture sequence. Although some form of motion sensing may be provided, the results are often disappointing. One approach that is commonly used for motion sensing is based on image processing. Although sensing of motion by analyzing image content can provide some measure of control over the image acquisition sequence, considerable data processing resources can be required for this purpose. Even where this is provided, however, it can be difficult for the analysis software to detect motion based on image content with a high degree of accuracy when the surrounding surface texture is relatively smooth, as can be true within the mouth.

JP 2012 075 690 relates to an intraoral camera having an acceleration sensor in a body case. A controller includes a body position monitor part connected to the acceleration sensor and an illumination control part connected to a high luminance LED. When the controller shifts an imaging device to an image recording mode, the illumination control part sets the high luminance LED to a low illuminance for recognizing a power source. Then, when the body position monitor part decides that the position of an intraoral insert part of an end side of the body case is lower than the position of a rear end side of the body case, the illumination control part changes the high luminance LED to a high illuminance for picking-up an image lighter than the low illuminance for recognizing the power source.

It would be advantageous to have an apparatus and method for detecting when the camera is moving and when it is at rest and for automatically initiating an image projection and acquisition sequence, while at the same time monitoring camera movement during the sequence to help determine whether or not the images are suitable for 3D model reconstruction.

### SUMMARY OF THE INVENTION

The present invention is directed to intra-oral image capture. Embodiments relate to triggering image projection and capture for 3D imaging in intra-oral applications, providing a method and apparatus for enabling image projection and capture according to sensed motion of the intra-oral camera.

It is a feature of the present invention that it initiates projection and capture of a sequence of images for 3D model reconstruction when the camera motion has stopped and monitors camera movement during the image capture sequence. Advantages of embodiments of the present invention include the capability for improved tracking of camera position and orientation for providing useful information for subsequent image stitching to provide 3D model data for display and other purposes.

According to an embodiment of the present invention, there is provided an intra-oral camera as set forth in claim 1 and a method for intra-oral imaging as set forth in claim 8. Further embodiments are inter alia disclosed in the respective dependent claims. The Camera inter alia comprises: a projector that is energizable to emit patterned illumination; an imaging sensor that is energizable to obtain image content; an inertial motion sensing element (motion sensor, motion sensing element or motion sensing device) that provides signals indicative of acceleration of the intra-oral imaging apparatus along at least one axis; and a processor that is in signal communication with the projector, the imaging sensor, and the inertial motion sensing element and is configured to initiate image acquisition according to signals obtained from the inertial motion sensing element.

The method comprises: detecting inertial energy of an intra-oral camera along at least one axis; initiating an imaging sequence when the detected inertial energy is below a first predetermined threshold, wherein the imaging sequence comprises projecting and acquiring image data for one or more projected patterned-light images; and processing the acquired image data and displaying a three-dimensional model according to the processed image data.

These objects are given only by way of illustrative example, and such objects may be exemplary of one or more embodiments of the invention. Other desirable objectives and advantages inherently achieved by the disclosed invention may occur or become apparent to those skilled in the art. The invention is defined by the appended claims.

The foregoing and other objects, features, and advantages of the invention will be apparent from the following more particular description of the embodiments of the invention, as illustrated in the accompanying drawings. The elements of the drawings are not necessarily to scale relative to each other.
FIG. 1 is a schematic block diagram that shows components of an intra-oral imaging system for 3D imaging.
FIG. 2 is a schematic block diagram showing a signal processing sequence for triggering and monitoring the image capture sequence according to an embodiment of the present invention.
FIG. 3 is a graph that plots typical energy and timing for an intra-oral camera that is at rest or is moving.
FIG. 4 is a logic flow diagram that shows steps in an imaging sequence using inertial energy detection.

### DETAILED DESCRIPTION OF THE INVENTION

Figures provided herein are given in order to illustrate key principles of operation and component relationships along their respective optical paths according to the present invention and are not drawn with intent to show actual size or scale. Some exaggeration may be necessary in order to emphasize basic structural relationships or principles of operation. Some conventional components that would be needed for implementation of the described embodiments, such as support components used for providing power, for packaging, and for mounting, for example, are not shown in the drawings in order to simplify description of the invention itself. In the drawings and text that follow, like components are designated with like reference numerals, and similar descriptions concerning components and arrangement or interaction of components already described are omitted.

In the context of the present disclosure, the terms "user", "technician", "practitioner", and "operator" are considered to be equivalent and refer to the person who operates the intra-oral imaging system and views its results.

In the context of the present invention, the term "energizable" describes a component or device that is enabled to perform a function upon receiving power and, optionally, upon also receiving an enabling signal. An image sensor, for example, is energizable to record image data when it receives the necessary power and enablement signals.

In the context of the present invention, an imaging sequence for an intra-oral imaging system that provides a 3D surface comprises projecting and detecting patterned illumination that comprises one or more 2D patterned-light images, typically a plurality of monochrome patterned-light images in sequence, and optionally detecting one or more color images that are then used to reconstruct the image volume. The patterned-light images can include a set of fringe pattern images or other images used for patterned light profiling of various types, for example. See for example, US 2010/0268069 (Liang) or PCT/US12/52178, both of which are incorporated herein by reference in their entirety.

In intra-oral imaging and other imaging applications, 3D imaging is accomplished by obtaining the sequence of 2D images illuminated by the patterned light and using the 2D image content to reconstruct the 3D surface for display. In order to perform the needed reconstruction, the 2D images are registered to each other, so that the same pixel content in two or more images of the patterned-light sequence can be identified. One difficulty in obtaining the 2D image sequence relates to camera movement. When the camera is being moved from one position to another, the needed image registration can be difficult to ascertain and the obtained images can have blurring or other motion-related artifacts. Thus, there is little or no value in obtaining images during camera movement. Once the camera is at rest, or substantially at rest, such as when placed and held in a specific position in the patient's mouth, then the sequence of images can be obtained.

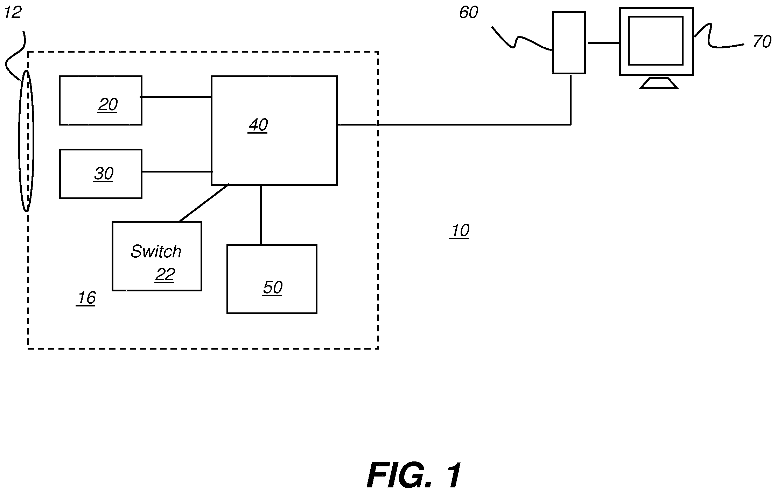

As noted in the background section given previously, conventional imaging devices have used various methods for motion detection, including image analysis. The motion-sensing results are then used to provide image stabilization. By contrast to conventional approaches, embodiments of the present invention use an inertial sensor to trigger an image capture sequence once motion has substantially stopped and further track inertial sensor output during the image capture sequence. Embodiments of the present invention address the problems of initiating the imaging sequence and monitoring camera movement during imaging by sensing signals related to camera acceleration along one or more axes. The schematic block diagram of Figure 1 shows components of an intra-oral imaging system 10 for 3D imaging. An intra-oral camera 16 has the components shown within the dashed box outline and typically also has additional components, such as power supply, battery, or wireless communication components, for example. System optics 12 includes one or more lenses for directing illumination to the tooth or other structure and for obtaining the image content therefrom. A projector 20 provides illumination, such as by projecting a light pattern or other patterned illumination onto the surface of the tooth or structure. According to an embodiment of the present invention, the projected illumination pattern is a fringe pattern, with an arrangement of lines of light that can be imaged and analyzed to show how the underlying surface is structured. Following pattern projection, illumination for obtaining a full 2D image is provided from projector 20 or from some other light source. An image sensor 30 provides an array of image sensing devices that obtain the image-bearing light and provide the image content to a processor 40. Image sensor 30 may be a CCD (Charge-Coupled Device) or CMOS (Complementary Metal Oxide Semiconductor) imaging array, for example. Processor 40 can be a dedicated processor or microprocessor, for example, and can provide some measure of processing for the image data. In the embodiment shown in Figure 1, processor 40 is in signal communication with an external computer 60 and a display 70 for displaying the reconstructed 3D image.

Still referring to Figure 1, an Inertial Measurement Unit (IMU) acts as a motion sensing element 50 and is in signal communication with processor 40 and provides the needed sensing to indicate movement of the camera. Motion sensing element 50 senses motion relative to at least one of the orthogonal axes, such as x, y, or z. Element 50 then provides signal output with one or more signals indicative of this movement to processor 40.

It is noted that commercially available IMU devices can include devices such as accelerometers for different axes as well as having gyroscopic, magnetic, and other sensors. However, acceleration or other motion-sensing along at least one axis or two mutually orthogonal axes provides sufficient information for control of the imaging sequence for the purpose of imaging sequence initiation and monitoring, as described herein. The accelerometer output is a signal that is indicative of static acceleration, such as due to gravity, and dynamic acceleration from hand vibration and from hand and arm movement of the operator. Inertial sensing can be provided from a Micro-ElectroMechanical System or MEMS device that is used as inertial motion sensing element 50, for example.

An optional switch 22 is provided, in communication with processor 40, for manual and/or automated enabling or interruption of the imaging sequence. A manually operated switch 22 can allow the operator to interrupt or temporarily disable image capture until the camera 16 is properly positioned within the mouth of the patient. An automated switch 22 can monitor the status of camera 16, such as monitoring position and orientation with respect to previous image captures, so that image combination can be correctly performed, for example.

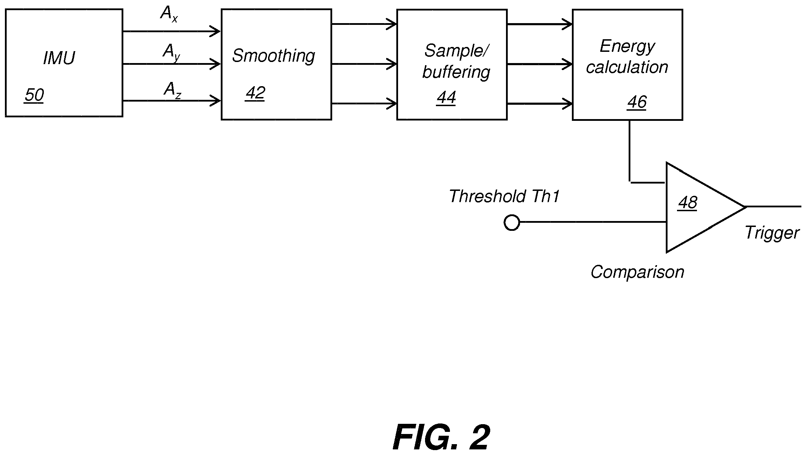

The schematic block diagram of Figure 2 shows how the signals provided by IMU 50 are processed for initiating and controlling image acquisition. When the intra-oral camera 16 (Figure 1) is moved by the technician, there is increased signal activity with respect to at least one of the orthogonal axes, with corresponding signals Aₓ, A_{y}, and A_{z} (Figure 2). An optional smoothing filter 42 provides smoothing and averaging of the received signal. A buffer 44 then buffers the received signals for processing; the signals are sampled a number of times (N) at a suitable frequency and duration (or "window") for measuring inertial movement of the camera. An energy calculation sequence 46 then calculates how much signal activity is sensed for the corresponding signal. Comparison against a threshold value Th1 is then performed, as represented in the schematic of Figure 2 by a comparator 48. When the analyzed signals A₁, A₂, and A₃ indicate that the camera is substantially at rest, a trigger signal is provided to initiate image acquisition. The circuitry and functions of filter 42, buffer 44, calculation sequence 46, and comparator 48 can be performed at processor 40 or using components that support the processor 40 function. Switch 22 (Figure 1) can override or enable the trigger signal provided by the signal processing shown in Figure 2.

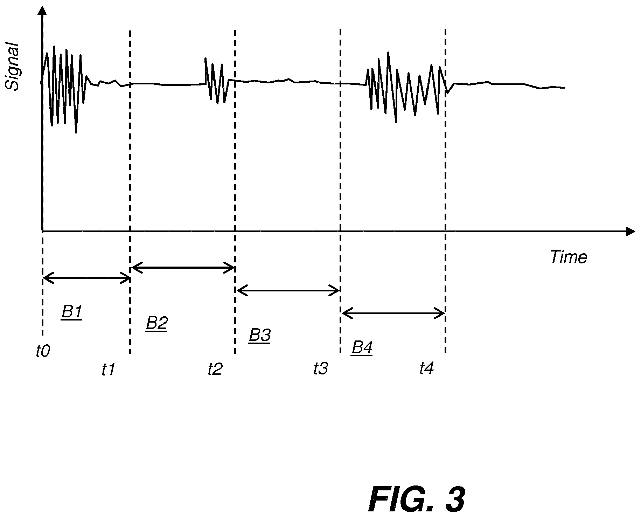

The graph of Figure 3 shows schematically, for the signal related to a single axis, aspects of image acquisition and monitoring with sample times and intervals to illustrate how image acquisition and monitoring operate according to an embodiment of the present invention. At a time t0, the signal level shows significant activity. Thus, during an interval B1 between time t0 and time t1, no images are obtained. At time t1, the signal level shows that the camera is substantially at rest (stable); after a suitable time interval that verifies the "at rest" status to help eliminate false positives, the image acquisition sequence is triggered. During an interval B2 between time t1 and a time t2, images are acquired. Since monitoring also occurs during interval B2, some motion is detected. However, this amount of motion may be negligible, so that the images obtained can still be retained and effectively used for generating volume data. At time t2, the camera is determined to be substantially at rest and image acquisition is triggered. This imaging interval B3 shows relatively no movement of the camera. At a time t3, the image acquisition sequence is triggered and images are acquired during an interval B4. However, significant movement during interval B4 is detected; the obtained images may not be used for reconstruction of the 3D volume image where this is the case.

Energy calculation sequence 46 in Figure 2 quantifies the relative amount of signal activity for the different axes Aₓ, A_{y}, and A_{z} and can be executed in any of a number of ways. The metric for measuring signal activity, and thus movement, can be computed using any of the following, for example:
(i) Average Energy of the output of "N" sample windows.
(ii) Variance of the N sample windows.
(iii) Mean of the N sample windows.

The window duration and sampling frequency can be fixed or variable, such as modified by the user, for example.

Where a 3-axis IMU is provided, a 3-component vector can be formed from the values for each axis. The norm of this vector is obtained by summing the squares of the axis values, then taking the square root of the result. This calculated norm is then compared against a threshold (Th1) value to determine whether or not an imaging sequence can be initiated. With this type of calculation, motion along any axis in the system can be detected.

The activity is continuously monitored in the background at a sufficient sampling frequency. The sampling frequency of monitoring and the window length N determine the responsiveness of the system.

Since there is always some inherent noise in the accelerometer output, the measured activity is non-zero. Threshold Th1 is predetermined but can be varied to adjust sensitivity to sensed movement. If activity is less than threshold Th1, the device is said to be at rest; otherwise it is in motion. This condition, however, may not always be true. The device can be intermittently moving, for example. It may not be possible to decide if a device is still or not based on measurement from a single time instance. Instead, after obtaining the first "device stable" output, the system monitors it for a time T mS and makes sure that the position is indeed stable for this entire duration before initiating a capture. This helps avoid false triggers and to minimize motion artifacts in the captured images. T is predetermined but can be changed to adjust sensitivity to sensed movement.

According to an embodiment of the present invention, there can be different image capture modes depending, in part, on how the image data is correlated to 3D data that is obtained from analyzing results from pattern projection. Various types of continuous or discrete imaging modes can be used. In one type of continuous imaging mode, intra-oral imaging system 10 triggers an image acquisition whenever it detects that intra-oral camera 16 is stationary. In another continuous imaging mode, system 10 triggers a new image capture periodically, at predetermined intervals. Captured images are processed continuously in the background to provide the needed 3D surface data. In a discrete imaging mode, the intra-oral imaging system 10 triggers an image acquisition whenever it detects that intra-oral camera 16 is stationary and 3D information reconstructed from the captured image data is updated.

In either continuous or discrete capture modes, intra-oral camera 16 can obtain feedback data to control or interrupt image capture using switch 22 (Figure 1), which can be implemented either in hardware or software. This feedback can be used to stop, start, or interrupt the auto-capture system.

It is noted that other timing sequences can be used, with image acquisition initiated and controlled according to movement data from the intra-oral camera.

Referring to Figure 1, the processing and display functions for 3D imaging are performed by external computer 60 rather than by processor 40. It can be appreciated that a significant amount of processing can be performed by processor 40; however, it is generally more convenient to provide image and data processing at computer 60, apart from intra oral camera 16.

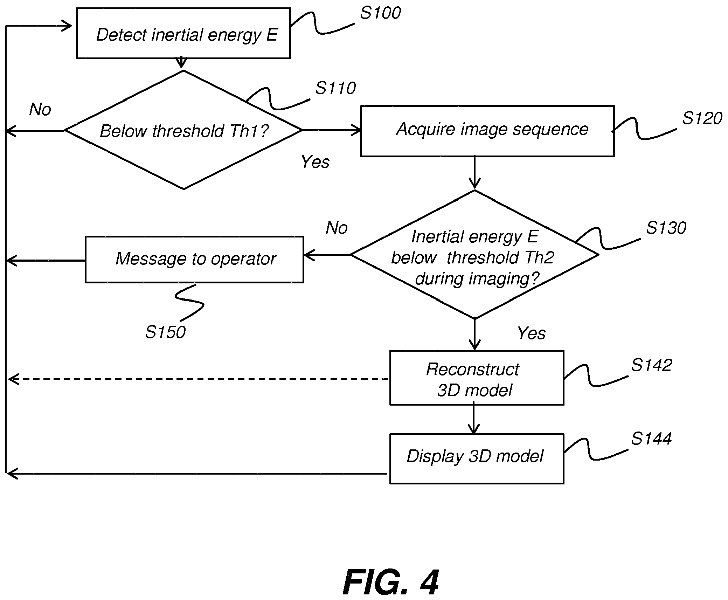

The logic flow diagram of Figure 4 shows steps in an imaging sequence for obtaining a 3D model using the apparatus and methods of an embodiment of the present invention. In a motion detection step S100, motion sensing element 50 and related logic detect inertial energy of intra-oral camera 16 along at least one axis. A threshold test step S1 10 is executed to detect when the inertial energy is below a predetermined threshold Th1 for a sufficient time. When this is the case, an imaging sequence is initiated in an acquire images step S120. If the inertial energy exceeds the threshold, monitoring continues in motion detection step S100. An optional monitoring and test step S130 monitors the inertial energy of the camera during the imaging sequence. If this energy is excessive, as described with respect to interval B4 in Figure 3, an optional warning step S150 executes and the operator is provided with a warning message or audible or visual signal that indicates that the image data may not be suitable due to movement above a second predetermined threshold Th2. Monitoring and test step S130 may assess the inertial energy using the same threshold Th1 used for initiation of the imaging sequence or may apply a different threshold or reference measurement. Alternately, the imaging sequence is terminated and no results are provided when excessive movement during imaging is detected. At the end of processing, or refreshed continually at intervals during image acquisition and processing, a reconstruction step S142 performs 3D model reconstruction according to the obtained 2D images. A display step S144 then displays results of the 3D reconstruction process. Steps S142 and S144 can be performed in parallel with ongoing image acquisition with constant refresh of the display or at termination of the image acquisition process.

The invention has been described in detail with particular reference to a presently preferred embodiment, but it will be understood that variations and modifications can be effected within the spirit and scope of the invention. For example, any of a number of different types of spatial light modulator could be used as part of the fringe pattern generator. Thresholds used for energy calculation and monitoring can be set as part of a calibration procedure and may default to factory settings or be set to values entered by the operator at a particular site. The presently disclosed embodiments are therefore considered in all respects to be illustrative and not restrictive. The scope of the invention is indicated by the appended claims, and all changes that come within the meaning and range of equivalents thereof are intended to be embraced therein.

## Claims

1. An intra-oral camera (16) comprising:
a projector (20) energizable to emit patterned illumination;
an imaging sensor (30) energizable to obtain image content;
an inertial motion sensing element (50) that provides one or more signals indicative of acceleration of the intra-oral imaging apparatus along at least one axis; and
a processor (40) in signal communication with the projector (20), the imaging sensor (30), and the inertial motion sensing element (50), wherein the processor (40) is configured to initiate image acquisition according to the one or more signals obtained from the inertial motion sensing element (50) when the detected inertial energy of the intra oral camera (16) along at least one axis is below a predetermined threshold.

2. The intra-oral camera (16) of claim 1 further comprising a display (70) that is in signal communication with the processor (40) for displaying a three-dimensional model formed according to the obtained image content.

3. The intra-oral camera (16) of claim 1 further comprising a switch (22) that is in signal communication with the processor (40) for interrupting image acquisition.

4. The intra-oral camera (16) of claim 1 wherein the inertial motion sensing element (50) further comprises one or more of a gyroscope and a magnetometer.

5. The intra-oral camera of claim 3 wherein the switch (22) is a manual switch.

6. The intra-oral camera of claim 1 wherein the inertial motion sensing element (50) provides the one or more signals indicative of motion along any of three orthogonal axes.

7. The intra-oral camera (16) of claim 1, wherein:
the inertial motion sensing element (50) provides the one or more signals indicative of acceleration of the intra-oral imaging apparatus along at least two orthogonal axes; and
the processor (40) is an external computer (60).

8. A method for intra-oral imaging, the method comprising:
detecting inertial energy of an intra-oral camera (16) along at least one axis from an inertial motion sensing element (50);
initiating by a processor (40) an imaging sequence when the detected inertial energy is below a first predetermined threshold, wherein the imaging sequence comprises projecting and acquiring image data for one or more projected patterned-light images; and
processing by the processor (40) the acquired image data and displaying a three-dimensional model according to the processed image data.

9. The method of claim 8 further comprising monitoring the inertial energy of the camera (16) during the imaging sequence and indicating a level of movement during imaging that exceeds a second predetermined threshold.

10. The method of claim 8 wherein the projected patterned-light images comprise one or more fringe pattern images.

11. The method of claim 8 wherein detecting inertial energy comprises obtaining one or more signals from an accelerometer.

12. The method of claim 8 wherein detecting inertial energy comprises averaging the energy of one or more signals from a motion sensor.

13. The method of claim 8 wherein detecting inertial energy comprises computing the variance of the energy of one or more signals from a motion sensor.

## Patentansprüche

1. Intraorale Kamera (16), die Folgendes aufweist:
einen Projektor (20), der erregbar ist zum Emittieren gemusterter Beleuchtung;
einen Abbildungssensor (30), der erregbar ist zum Erhalten eines Bildinhalts; ein Trägheitsbewegungssensorelement (50), das ein oder mehrere Signal/e vorsieht, die eine Beschleunigung der intraoralen Abbildungsvorrichtung entlang wenigstens einer Achse angeben; und
einen Prozessor (40) in Signalkommunikation mit dem Projektor (20), dem Abbildungssensor (30) und dem Trägheitsbewegungssensorelement (50), wobei der Prozessor (40) konfiguriert ist zum Initiieren einer Bilderfassung gemäß dem einen oder den mehreren Signalen, die von dem Trägheitsbewegungssensorelement (50) erhalten werden, wenn die detektierte Trägheitsenergie der intraoralen Kamera (16) entlang wenigstens einer Achse unter einen vorbestimmten Schwellenwert liegt.

2. Intraorale Kamera (16) nach Anspruch 1, die ferner eine Anzeige (70) aufweist, welche in Signalkommunikation mit dem Prozessor (40) zum Anzeigen eines gemäß dem erhaltenen Bildinhalt gebildeten dreidimensionalen Modells steht.

3. Intraorale Kamera (16) nach Anspruch 1, die ferner einen Schalter (22) aufweist, welcher mit dem Prozessor (40) zum Unterbrechen der Bilderfassung in Signalkommunikation steht.

4. Intraorale Kamera (16) nach Anspruch 1, wobei das Trägheitsbewegungssensorelement (50) ferner ein oder mehr eines Gyroskops und eines Magnetometers aufweist.

5. Intraorale Kamera nach Anspruch 3, wobei der Schalter (22) ein manueller Schalter ist.

6. Intraorale Kamera nach Anspruch 1, wobei das Trägheitsbewegungssensorelement (50) ein oder mehrere Signal/e vorsieht, die Bewegung entlang einer der drei orthogonalen Achsen angibt.

7. Intraorale Kamera (16) nach Anspruch 1, wobei:
das Trägheitsbewegungssensorelement (50) das eine oder die mehreren Signal/e vorsieht, die die Beschleunigung der intraoralen Abbildungsvorrichtung entlang wenigstens zweier orthogonaler Achsen anzeigt; und
der Prozessor (40) ein externer Computer (60) ist.

8. Verfahren zur intraoralen Abbildung, wobei das Verfahren Folgendes aufweist:
Detektieren einer Trägheitsenergie einer intraoralen Kamera (16) entlang wenigstens einer Achse von einem Trägheitsbewegungssensorelement (50);
Initiieren, durch einen Prozessor (40), einer Abbildungssequenz, wenn die detektierte Trägheitsenergie unter einem ersten vorbestimmten Schwellenwert liegt, wobei die Abbildungssequenz ein Projizieren und Erfassen von Bilddaten für ein oder mehr projizierte gemusterte Lichtbilder aufweist; und
Verarbeiten, durch den Prozessor (40), der erfassten Bilddaten und Anzeigen eines dreidimensionalen Modells gemäß den verarbeiteten Bilddaten.

9. Verfahren nach Anspruch 8, das ferner ein Überwachen der Trägheitsenergie der Kamera (16) während der Abbildungssequenz aufweist, und ein Bewegungspegel während der Abbildung angibt, welches einen zweiten vorbestimmten Schwellenwert übersteigt.

10. Verfahren nach Anspruch 8, wobei die projizierten gemusterten Lichtbilder ein oder mehr Streifenmusterbild/er aufweisen.

11. Verfahren nach Anspruch 8, wobei das Detektieren der Trägheitsenergie das Erhalten eines oder mehrerer Signal/e von einem Beschleunigungsmesser aufweist.

12. Verfahren nach Anspruch 8, wobei das Detektieren der Trägheitsenergie das Mitteln der Energie von einem oder mehrerer Signal/e von einem Bewegungssensor aufweist.

13. Verfahren nach Anspruch 8, wobei das Detektieren der Trägheitsenergie das Berechnen der Energieabweichung von einem oder mehreren Signal/en von einem Bewegungssensor aufweist.

## Revendications

1. Caméra intra-orale (16) comprenant :
un projecteur (20) pouvant être activé pour émettre un éclairage à motif ;
un capteur d'image (30) pouvant être activé pour obtenir un contenu d'image ;
un élément de détection de mouvement inertiel (50) qui fournit un ou plusieurs signaux indiquant l'accélération de l'appareil d'imagerie intra-orale le long d'au moins un axe ; et
un processeur (40) en communication de signal avec le projecteur (20), le capteur d'image (30) et l'élément de détection de mouvement inertiel (50), dans lequel le processeur (40) est configuré pour déclencher une acquisition d'image en fonction du ou des signaux obtenus à partir de l'élément de détection de mouvement inertiel (50) lorsque l'énergie inertiel détectée de la caméra intra-orale (16) le long d'au moins un axe est inférieure à un seuil prédéterminé.

2. Caméra intra-orale (16) selon la revendication 1, comprenant en outre un affichage (70) en communication de signal avec le processeur (40) pour afficher un modèle tridimensionnel formé selon le contenu d'image obtenu.

3. Caméra intra-orale (16) selon la revendication 1, comprenant en outre un commutateur (22) qui est en communication de signal avec le processeur (40) pour interrompre l'acquisition d'image.

4. Caméra intra-orale (16) selon la revendication 1, dans laquelle le capteur de mouvement inertiel (50) comprend en outre un ou plusieurs parmi un gyroscope et un magnétomètre.

5. Caméra intra-orale selon la revendication 3, dans laquelle le commutateur (22) est un commutateur manuel.

6. Caméra intra-orale selon la revendication 1, dans laquelle le capteur de mouvement inertiel (50) fournit le ou les signaux indicatifs du mouvement le long de l'un quelconque de trois axes orthogonaux.

7. Caméra intra-orale (16) dans lequel :
l'élément de détection de mouvement inertiel (50) fournit le ou les signaux indiquant l'accélération de l'appareil d'imagerie intra-orale selon au moins deux axes orthogonaux ; et
le processeur (40) est un ordinateur externe (60).

8. Procédé d'imagerie intra-orale, le procédé comprenant :
la détection de l'énergie inertielle d'une caméra intra-orale (16) le long d'au moins un axe d'un élément de détection de mouvement inertiel (50) ;
l'initialisation, par un processeur (40), d'une séquence d'imagerie lorsque l'énergie inertielle détectée est inférieure à un premier seuil prédéterminé, dans lequel la séquence d'imagerie comprend la projection et l'acquisition de données d'image pour une ou plusieurs images d'éclairage à motif projetées ; et
le traitement, par le processeur (40), des données d'image acquises et l'affichage d'un modèle tridimensionnel en fonction des données d'image traitées.

9. Procédé selon la revendication 8, comprenant en outre la surveillance de l'énergie inertielle de la caméra (16) pendant la séquence d'imagerie et l'indication d'un niveau de mouvement pendant l'imagerie qui dépasse un deuxième seuil prédéterminé.

10. Procédé selon la revendication 8, dans lequel les images d'éclairage à motif projetées comprennent une ou plusieurs images à motif de franges.

11. Procédé selon la revendication 8, dans lequel la détection d'énergie inertielle comprend l'obtention d'un ou plusieurs signaux d'un accéléromètre.

12. Procédé selon la revendication 8, dans lequel la détection d'énergie inertielle comprend la mise en moyenne de l'énergie d'un ou de plusieurs signaux provenant d'un capteur de mouvement.

13. Procédé selon la revendication 8, dans lequel la détection d'énergie inertielle comprend le calcul de la variance de l'énergie d'un ou de plusieurs signaux provenant d'un capteur de mouvement.
